# EUROPÄISCHE PATENTSCHRIFT

(11) **EP 4 180 440 B1**
(45) Veröffentlichungstag und Bekanntmachung des Hinweises auf die Patenterteilung: **28.01.2026**
(21) Anmeldenummer: 21208544.3
(22) Anmeldetag: 16.11.2021
(51) Int. Cl.: C07F 9/6596, C07F 15/00, B01J 31/24, C07D 215/38, C07D 401/04, C07D 403/04, C07D 413/04, B01J 31/16

(54) **CYCLISCHE BIARYLPHOSPHINE ALS LIGANDEN FÜR PALLADIUMHALTIGE KATALYSATOREN IN KREUZKUPPLUNGSREAKTIONEN**
CYCLIC BIARYLPHOSPHINES AS LIGANDS FOR PALLADIUM-CONTAINING CATALYSTS IN CROSS-COUPLING REACTIONS
BIARYLPHOSPHINES CYCLIQUES COMME LIGANDS POUR CATALYSEURS CONTENANT DU PALLADIUM DANS LES RÉACTIONS DE COUPLAGE CROISÉ

(43) Veröffentlichungstag der Anmeldung: 17.05.2023
(73) Patentinhaber: Heraeus Precious Metals GmbH & Co. KG, 63450 Hanau (DE)
(72) Erfinder: GOCK, Michael, 63450 Hanau (DE); KRÄH, Sabrina, 81377 München (DE); TRAPP, Oliver, 81377 München (DE)
(74) Vertreter: Maiwald GmbH

(56) Entgegenhaltungen:
- US-A1- 2014 371 446
- YA A VERESHCHAGINA ET AL: "Conformational analysis of 1,4-heterophosphinanes", RUSSIAN JOURNAL OF GENERAL CHEMISTRY, NAUKA/INTERPERIODICA, MO, vol. 77, no. 1, 1 January 2007 (2007-01-01), pages 36 - 39, XP019484190, ISSN: 1608-3350, DOI: 10.1134/S1070363207010057

## Beschreibung

Die vorliegende Erfindung betrifft cyclische Biarylphosphine und deren Verwendung als Liganden in Palladiumkomplexen bei palladiumkatalysierten Kupplungsreaktionen (wie z.B. der Buchwald-Hartwig-Kupplung).

Unter einer Kreuzkupplung wird eine Kupplungsreaktion verstanden, bei der zwei unterschiedliche Moleküle in Anwesenheit eines geeigneten Katalysators unter Ausbildung einer Kohlenstoff-Kohlenstoff- oder Kohlenstoff-Heteroatom-Bindung miteinander reagieren. Von großer praktischer Bedeutung (z.B. bei der Synthese pharmazeutischer Wirkstoffe) sind insbesondere palladiumkatalysierte Kreuzkupplungen. Beispielhaft können in diesem Zusammenhang die Stille-Kupplung, Suzuki-Kupplung (auch als Suzuki-Miyaura-Kupplung bekannt) oder Negishi-Kupplung genannt werden. Im Jahr 2010 erhielten R.F. Heck, E. Negishi und A. Suzuki den Nobelpreis für Chemie für ihre Arbeiten zu Kupplungsreaktionen.

In den folgenden Übersichtsartikeln wird die Verwendung von Kreuzkupplungsreaktionen bei der Synthese pharmazeutischer Wirkstoffe eingehender beschrieben:
- H.C. Shen, "Selected Applications of Transition Metal-Catalyzed Carbon-Carbon Cross-Coupling Reactions in the Pharmaceutical Industry", S. 25-96, in "Applications of Transition Metal Catalysis in Drug Discovery and Development: An Industrial Perspective", Ed.: M.L. Crawley and B.M. Trost, 2012, John Wiley & Sons;
- Q. Gu et al., "Palladium catalyzed C-C and C-N bond forming reactions: An update on the synthesis of pharmaceuticals from 2015-2020", Org. Chem. Front., 2021, 8, S. 384-414.

Eine weitere dem Fachmann bekannte Kupplungsreaktion ist die Buchwald-Hartwig-Kupplung, bei der ein Aryl- oder Heteroarylhalogenid oder -pseudohalogenid und ein primäres oder sekundäres Amin in Anwesenheit einer Base und eines palladiumhaltigen Katalysators unter Ausbildung einer C-N-Bindung miteinander umgesetzt werden.

In den folgenden Übersichtsartikeln wird der aktuelle Stand auf dem Gebiet der C-N-Kupplungsreaktionen zusammengefasst:
- R. Dorel et al., "The Buchwald-Hartwig Amination After 25 Years", Angew. Chem. Int. Ed., 2019, 58, S. 17118-17129;
- S.L. Buchwald et al., "Dialkylbiaryl phosphines in Pd-catalyzed amination: a user's guide", Chem. Sci., 2011, 2, S. 27-50;
- S.L. Buchwald et al., "Biaryl Phosphane Ligands in Palladium-Catalyzed Amination", Angew. Chem. Int. Ed., 2008, 47, S. 6338-6361;
- S.L. Buchwald et al., "Applications of Palladium-Catalyzed C-N Cross-Coupling Reactions", Chem. Rev., 2016 116, S. 12564-12649.

Übliche Katalysatoren auf dem Gebiet der Kupplungsreaktionen sind Palladiumkomplexe, die einen oder mehrere Phosphinliganden und optional noch weitere Liganden enthalten. Diese Pd-Komplexe mit geeigneten Phosphinliganden können vorab hergestellt und bis zu ihrer Verwendung gelagert oder alternativ *in situ* während der zu katalysierenden Reaktion generiert werden (z.B. durch getrennte Zugabe eines Palladiumsalzes und des Phosphins zum Reaktionsmedium, so dass die Ausbildung eines phosphinhaltigen Pd-Komplexes erst im Reaktionsmedium erfolgt).

Es ist bekannt, dass nicht-cyclische Biarylphosphine als Liganden für Pd-Komplexe in palladiumkatalysierten Kupplungsreaktionen (wie z.B. der Buchwald-Hartwig-Kupplung) fungieren können, siehe z.B. S.L. Buchwald et al., "Dialkylbiaryl phosphines in Pd-catalyzed amination: a user's guide", Chem. Sci., 2011, 2, S. 27-50. In diesen nicht-cyclischen Phosphinen liegt kein phosphorhaltiger Ring (d.h. kein Ring, der Phosphor als Ringatom enthält) vor.

Es sind auch cyclische Biarylphosphine (d.h. Phosphine, in denen das Phosphoratom eines der ringbildenden Atome ist) als Liganden für Pd-Komplexe in palladiumkatalysierten Kupplungsreaktionen bekannt.

S. Shekhar et al., ACS Catal., 2019, 9, S. 11691-11708, und S. Shekhar et al., ACS Catal., 2020, 10, S. 15008-15018, beschreiben Biarylphosphorinane und deren Verwendung als Pd-Komplexliganden für palladiumkatalysierte Kupplungsreaktionen.
C. Maumela et al., RSC Adv., 2021, 11, S. 26883-26891, beschreiben Biarylphobane und Biarylphosphatrioxadamantane und deren Verwendung als Pd-Komplexliganden für palladiumkatalysierte Suzuki-Kupplungen.

WO 2012/009698 A1 beschreibt ein monocyclisches, bicyclisches oder tricyclisches Biarylphosphin, wobei das heterocyclische Ringsystem neben dem Phosphoratom noch vier Kohlenstoffatome und optional mindestens ein weiteres Ringatom, das aus Kohlenstoff, Sauerstoff, Stickstoff, Phosphor und Schwefel ausgewählt ist, enthält. US2014/371446 A1 offenbart Liganden, die für Komplexe in der Katalyse verwendet werden können.

Y.A. Vereshchagina et al., Russian Journal of General Chemistry, 2007, Band 77, Nr. 1, S. 36-39, beschreiben die Konformation unterschiedlicher Monoaryl-1,4-Heterophosphinane. Eines der 1,4-Heterophosphinane weist ein Siliziumatom als Ringatom auf. Mögliche Verwendungen der Verbindungen werden nicht erwähnt.

Die Ausbildung einer Kohlenstoff-Heteroatom-Bindung (insbesondere einer C-N-Bindung) über eine Kupplungsreaktion, insbesondere eine Buchwald-Hartwig-Kupplung, erfordert bei der Verwendung N-heterocyclischer Arylhalogenide üblicherweise relativ lange Reaktionszeiten. Sofern keine effizienteren Katalysatoren zur Verfügung stehen, kann beispielsweise die Reaktionstemperatur erhöht werden, um kürzere Reaktionszeiten zu realisieren. Dies führt aber üblicherweise zu unerwünschten Nebenreaktionen, die die Ausbeute des erwünschten Kupplungsprodukts weiter reduzieren.

Eine Aufgabe der vorliegenden Erfindung ist die Bereitstellung geeigneter Phosphine, die als Liganden in Palladiumkomplexen bei palladiumkatalysierten Kreuzkupplungsreaktionen verwendbar sind. Insbesondere sollten diese Phosphine als Liganden in Palladiumkomplexen auch dann effiziente C-N-Kupplungsreaktionen (z.B. in Form einer Buchwald-Hartwig-Kupplung) ermöglichen, wenn N-heterocyclische Arylhalogenide oder -pseudohalogenide als Reaktanten eingesetzt werden.

Gelöst wird die Aufgabe durch ein Phosphin der Formel (1) 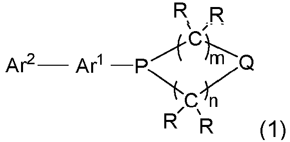 wobei
- Q SiR³R⁴ ist;
- m 1, 2 oder 3 ist; n 1, 2 oder 3 ist; unter der Maßgabe, dass folgende Beziehung erfüllt ist: 3 ≤ m + n ≤ 5;
- die Reste R unabhängig voneinander ein Wasserstoffatom oder ein C₁₋₄-Alkyl sind; oder zwei Reste R, die mit demselben Kohlenstoffatom verbunden sind, jeweils eine bivalente Alkylengruppe sind und zusammen mit dem Kohlenstoffatom, mit dem sie verbunden sind, einen 4- bis 7-gliedrigen (insbesondere 5- bis 6-gliedrigen) Ring bilden; oder zwei Reste R, die mit unterschiedlichen Kohlenstoffatomen verbunden sind, jeweils eine bivalente Alkylengruppe sind und zusammen mit den Kohlenstoffatomen, mit denen sie verbunden sind, einen 4- bis 7-gliedrigen (insbesondere 5- bis 6-gliedrigen) Ring bilden;
- Ar¹ ein Phenyl oder Naphthyl ist, das optional mit einer oder mehreren C₁₋₆-Alkylgruppen substituiert ist;
- Ar² ein Phenyl oder Naphthyl ist, das optional mit einer oder mehreren C₁₋₆-Alkylgruppen substituiert ist;
- R³ und R⁴ unabhängig voneinander ein C₁₋₄-Alkyl oder ein Phenyl, das optional mit einer oder mehreren C₁₋₆-Alkylgruppen substituiert ist, sind.

Die cyclischen Biarylphosphine der vorliegenden Erfindung enthalten in dem heterocyclischen Ring neben dem Phosphoratom noch ein weiteres Heteroatom als Ringatom, wobei dieses zusätzliche Heteroringatom Si ist. Wie nachfolgend noch eingehender beschrieben wird, ermöglicht die Verwendung des erfindungsgemäßen Phosphins als Ligand in Pd-Komplexen die Durchführung einer C-C-Kreuzkupplungsreaktion oder einer C-N-Kreuzkupplungsreaktion, beispielsweise der Buchwald-Hartwig-Kupplung, die bei relativ niedrigen Reaktionstemperaturen und relativ kurzen Reaktionszeiten zu hohen Ausbeuten führt, selbst wenn ein N-heterocyclisches Arylhalogenid oder -pseudohalogenid als Reaktant eingesetzt wird.

Der Palladiumkomplex, der das erfindungsgemäße Phosphin als einen Liganden enthält, kann bereits vor der zu katalysierenden Reaktion hergestellt und gegebenenfalls bis zu seiner Verwendung gelagert werden. Alternativ ist es auch möglich, dass das erfindungsgemäße Phosphin und eine als Präkursor fungierende Palladiumverbindung dem Reaktionsmedium zugegeben werden, so dass die Bildung eines Palladiumkomplexes, der das erfindungsgemäße Phosphin als einen Liganden enthält, *in situ* im Reaktionsmedium der Kupplungsreaktion erfolgt.

Wenn in der Formel (1) zwei Reste R, die mit demselben Kohlenstoffatom verbunden sind, zusammen mit diesem Kohlenstoffatom einen 4-bis 7-gliedrigen Ring bilden, liegt ein Spiro-Ringsystem vor.

Wenn in der Formel (1) zwei Reste R, die mit zwei benachbarten Kohlenstoffatomen verbunden sind, zusammen mit diesen benachbarten Kohlenstoffatomen einen 4- bis 7-gliedrigen Ring bilden, liegt ein anelliertes Ringsystem vor.

Wenn in der Formel (1) zwei Reste R, die mit zwei nicht benachbarten Kohlenstoffatomen verbunden sind, zusammen mit diesen nicht benachbarten Kohlenstoffatomen einen 4- bis 7-gliedrigen Ring bilden, liegt ein verbrücktes Ringsystem vor.

Bevorzugt weist das erfindungsgemäße Phosphin die folgende Formel (1a) auf: 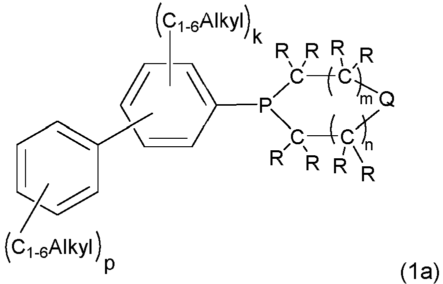 wobei
m 0 oder 1 (bevorzugter m=1) und n 1 oder 2 (bevorzugter n=1) sind;
k 0, 1, 2, 3 oder 4 ist;
p 0, 1, 2, 3, 4 oder 5 ist;
Q und R jeweils die oben angegebene Bedeutung aufweisen.

In einer bevorzugten Ausführungsform der vorliegenden Erfindung weist das Phosphin die folgende Formel (1b) auf: 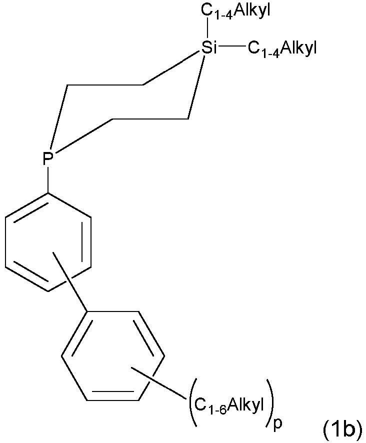 wobei
p 0, 1, 2, 3, 4 oder 5, bevorzugter 1, 2, 3 oder 4 ist.

Außerdem betrifft die vorliegende Erfindung ein Verfahren zur Herstellung des oben beschriebenen erfindungsgemäßen Phosphins, wobei ein Phosphin der Formel (5)

Ar¹-Ar²-PH₂ (5)

wobei
Ar¹ und Ar² jeweils die oben beschriebene Bedeutung aufweisen;
mit einer Base zumindest teilweise deprotoniert wird und das dabei erhaltene Phosphid umgesetzt wird mit einer Verbindung der Formel (6) 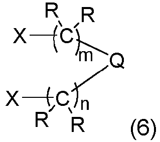 wobei
X ein Halogenatom (z.B. Cl, Br oder I) ist,
Q, R, m und n jeweils die obene beschriebene Bedeutung aufweisen.

Geeignete Basen, mit denen sich ein primäres Phosphin durch Deprotonierung in ein Phosphid überführen lässt, sind dem Fachmann bekannt. Beispielsweise ist die Base eine Organolithiumverbindung (z.B. Butyllithium).

Das Phosphin der Formel (5) kann beispielsweise erhalten werden, indem eine Verbindung der Formel (4) 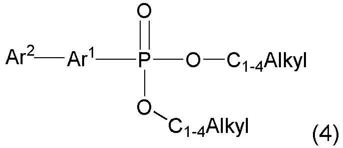 wobei
Ar¹ und Ar² jeweils die oben beschriebene Bedeutung aufweisen;
in Anwesenheit eines Reduktionsmittels zu dem Phosphin der Formel (5) reduziert wird.

Das Reduktionsmittel ist beispielsweise ein Metallhydrid (z.B. Lithiumaluminiumhydrid).

Wird mit dem erfindungsgemäßen Verfahren das Phosphin der obigen Formel (1a) hergestellt, erfolgt die Umsetzung des nach der Deprotonierung des Phosphins der Formel (5) erhaltenen Phosphids mit einer Verbindung der Formel (6a): 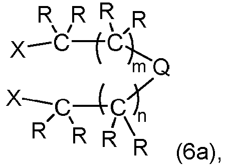 wobei
m 0 oder 1 (bevorzugter m=1) und n 1 oder 2 (bevorzugter n=1) sind;
X jeweils ein Halogenatom (z.B. Cl, Br oder I) ist;
Q und R jeweils die oben für Formel (1a) angegebene Bedeutung haben.

Wird mit dem erfindungsgemäßen Verfahren das Phosphin der obigen Formel (1b) hergestellt, erfolgt die Umsetzung des nach der Deprotonierung des Phosphins der Formel (5) erhaltenen Phosphids mit einer Verbindung der Formel (6b): 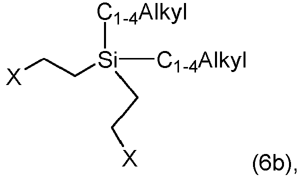 wobei X jeweils ein Halogenatom (z.B. Cl, Br oder I) ist.

Die Verbindung der Formel (4) kann beispielsweise hergestellt werden, indem eine Verbindung der Formel (2)

Ar¹-Ar²-X (2)

wobei
X ein Halogenatom (z.B. Cl, Br oder I) ist,
Ar¹ und Ar² jeweils die oben beschriebene Bedeutung aufweisen,
mit einer metallorganischen Verbindung, bevorzugt einer Organolithiumverbindung (z.B. Butyllithium), zu einem Zwischenprodukt umgesetzt wird und das Zwischenprodukt anschließend umgesetzt wird mit einer Verbindung der Formel (3)

O=P(O-C₁₋₄-Alkyl)₂X (3)

wobei
X ein Halogenatom (z.B. Cl, Br oder I) ist.

Außerdem betrifft die vorliegende Erfindung einen Palladiumkomplex, der als einen Liganden L¹ das oben beschriebene erfindungsgemäße Phosphin enthält.

Der Palladiumkomplex kann zusätzlich noch einen oder mehrere Liganden L² enthalten, die jeweils kein erfindungsgemäßes Phosphin sind. Geeignete Liganden für Palladiumkomplexe sind dem Fachmann bekannt.

Beispielsweise sind die weiteren Liganden L² des erfindungsgemäßen Pd-Komplexes unabhängig voneinander ausgewählt aus einem Halogenid (z.B. Cl⁻, Br⁻ oder I⁻); einem Aryl; einem Nitril (z.B. Acetonitril, Propionitril oder Benzonitril); einem Carboxylat (z.B. Acetat); einem konjugierten Dienon (z.B. ein 1,4-Dien-3-on wie Dibenzylidenaceton (dba)); einem Phosphin, das kein erfindungsgemäßes Phosphin ist (z.B. ein nicht-cyclisches Phosphin); einem Pseudohalogenid (z.B. CN⁻ oder OCN⁻) einem Amin oder Acetylacetonat.

Der Ligand L², der kein erfindungsgemäßes Phosphin ist, kann ein einzähniger oder alternativ ein mehrzähniger Ligand sein. Beispiele für solche mehrzähnigen Liganden umfassen Arylalkylamine oder Arylamine (z.B. Phenethylamin oder Naphthylamin) und Monoanionen davon.

Sofern der Ligand L² ein Phosphin ist, handelt es sich bevorzugt um ein nicht-cyclisches Phosphin, d.h. ein Phosphin, das keinen phosphorhaltigen Ring aufweist. Geeignete nicht-cyclische Phosphinliganden für Palladiumkomplexe sind dem Fachmann bekannt.

Bei dem Phosphinliganden L², der kein erfindungsgemäßes Phosphin ist, handelt es sich beispielsweise um ein Tri-C₁₋₆-Alkylphosphin, ein Tri-C₅₋₇-Cycloalkylphosphin oder ein Triarylphosphin (insbesondere ein Triphenylphosphin), wobei jede der Arylgruppen (die bevorzugt Phenylgruppen sind) optional durch eine oder mehrere C₁₋₄-Haloalkylgruppen (z.B. - CF₃), C₁₋₄-Alkylgruppen (z.B. Methyl) oder C₁₋₄ -Alkoxygruppen (z.B. Methoxy) substituiert ist.

Beispielsweise weist der Phosphinligand L² eine der folgenden Formeln (7.1), (7.2) oder (7.3) auf:

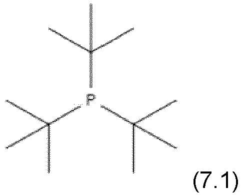

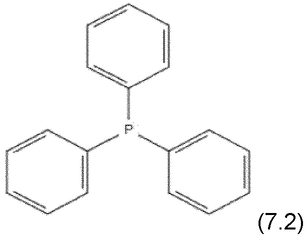

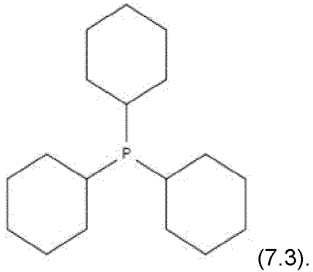

Alternativ kann es sich bei dem Phosphinliganden L² um ein Phosphin der folgenden Formel (8) handeln:

P(R¹)(R²)(R³) (8)

wobei
R¹ und R² unabhängig voneinander ausgewählt sind aus C₁₋₆-Alkyl and C₅₋₇-Cycloalkyl (z.B. Cyclohexyl),
R³ Biphenyl ist, das optional durch eine oder mehrere C₁₋₆-Alkylgruppen, C₁₋₆-Alkoxygruppen, Phenylgruppen oder Pyridylgruppen substituiert ist.

Beispielsweise weist der Phosphinligand L² eine der folgenden Formeln (8.1), (8.2), (8.3) oder (8.4) auf:

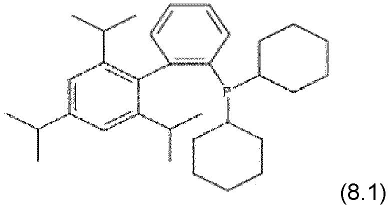

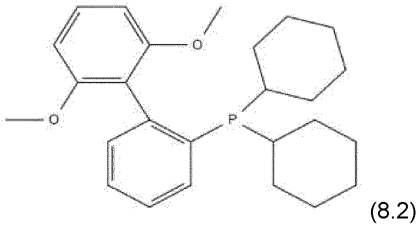

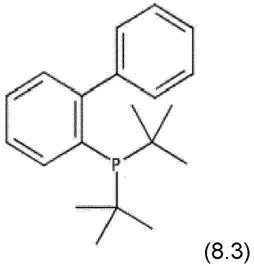

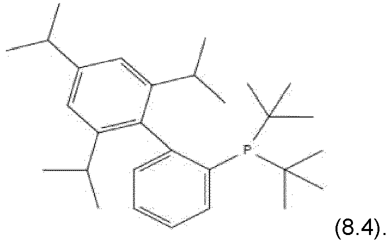

Der erfindungsgemäße Palladiumkomplex weist beispielsweise die folgende Formel (10) auf: 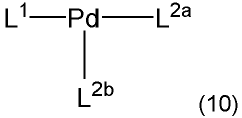 wobei
L¹ ein erfindungsgemäßes Phosphin ist,
L^{2a} und L^{2b} jeweils ein Ligand ist, der kein erfindungsgemäßes Phosphin ist.

Hinsichtlich geeigneter Liganden L^{2a} und L^{2b} kann auf die obigen Ausführungen zu dem Liganden L² verwiesen werden. Beispielsweise sind die Liganden L^{2a} und L^{2b} des erfindungsgemäßen Pd-Komplexes unabhängig voneinander ausgewählt aus einem Halogenid (z.B. Cl⁻, Br⁻ oder I⁻); einem Aryl; einem Nitril (z.B. Acetonitril, Propionitril oder Benzonitril); einem Carboxylat (z.B. Acetat); einem konjugierten Dienon (z.B. ein 1,4-Dien-3-on wie Dibenzylidenaceton (dba)); einem Phosphin, das kein erfindungsgemäßes Phosphin ist (z.B. ein nicht-cyclisches Phosphin); einem Pseudohalogenid (z.B. CN⁻ oder OCN⁻) einem Amin oder Acetylacetonat.

Ein beispielhafter Palladiumkomplex der vorliegenden Erfindung weist die folgende Formel (10.1) auf: 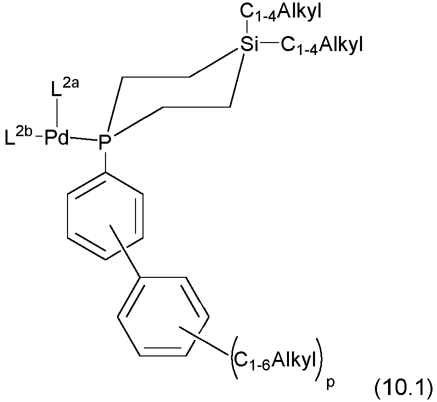
wobei die Liganden L^{2a} und L^{2b} die oben angegebenen Bedeutungen aufweisen
und p 0, 1, 2, 3 oder 4 ist.

Beispielsweise ist der Ligand L^{2a} Dibenzylidenaceton (dba) oder Acetonitril und der Ligand L^{2b} wird aus einem der oben für L² angegebenen Liganden ausgewählt.

Ein weiterer beispielhafter Palladiumkomplex der vorliegenden Erfindung weist die folgende Formel (10.2) auf: 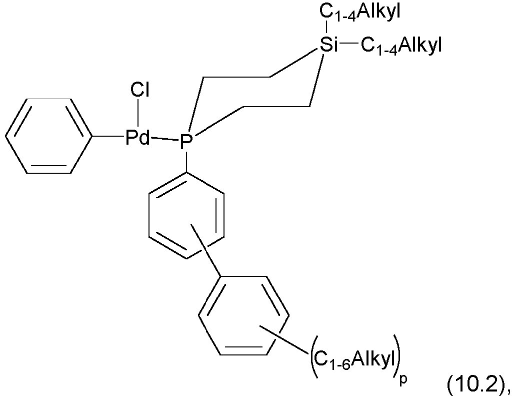 wobei p 0, 1, 2, 3 oder 4 ist.

Ein weiterer beispielhafter Palladiumkomplex der vorliegenden Erfindung weist die folgende Formel (10.3) auf: 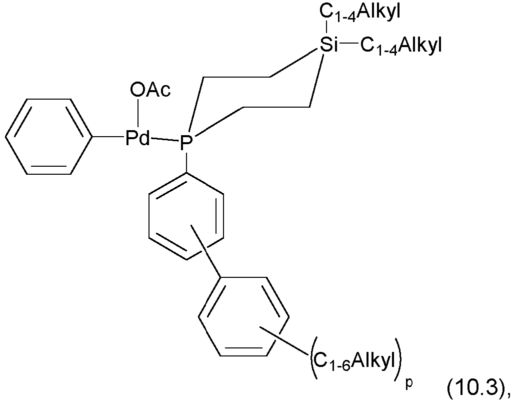 wobei p 0, 1, 2, 3 oder 4 ist.

Wie oben bereits erwähnt, können das erfindungsgemäße Phosphin und eine als Präkursor fungierende Palladiumverbindung, die das erfindungsgemäße Phosphin noch nicht enthält, dem Reaktionsmedium zugegeben werden, so dass die Bildung eines Palladiumkomplexes, der das erfindungsgemäße Phosphin als einen Liganden enthält, *in situ* im Reaktionsmedium der Kupplungsreaktion erfolgt.

Die vorliegende Erfindung betrifft daher auch eine Zusammensetzung, enthaltend
- eine Palladiumverbindung und
- das oben beschriebene erfindungsgemäße Phosphin.

Die Palladiumverbindung der erfindungsgemäßen Zusammensetzung enthält üblicherweise kein erfindungsgemäßes Phosphin.

Optional kann die Zusammensetzung in Form eines Kits, der die Palladiumverbindung und das erfindungsgemäße Phosphin in getrennten Behältern enthält, vorliegen.

Die Palladiumverbindung ist beispielsweise ein Palladiumsalz oder ein Palladiumkomplex, dessen Liganden kein erfindungsgemäßes Phosphin sind.

Das Palladiumsalz ist beispielsweise ein Palladiumacetat, ein Palladiumhalogenid (z.B. ein Palladiumchlorid, Palladiumbromid oder Palladiumiodid) oder ein Palladiumpseudohalogenid oder ein Gemisch aus mindestens zwei dieser Salze.

Handelt es sich bei der Palladiumverbindung um einen Palladiumkomplex, so sind dessen Liganden unabhängig voneinander beispielsweise ausgewählt aus einem Halogenid (z.B. Cl⁻, Br⁻ oder I⁻); einem Phosphin, das kein erfindungsgemäßes Phosphin ist (z.B. ein nicht-cyclisches Phosphin); einem konjugierten Dienon (z.B. ein 1,4-Dien-3-on wie Dibenzylidenaceton (dba)); einem Polyen (beispielsweise ein Polyen mit 2-4 Doppelbindungen, bevorzugt ein cyclisches Polyen, insbesondere ein cyclisches mit 2-4 Doppelbindungen wie z.B. Cyclooctadien oder Cyclooctatetraen); einem Nitril (z.B. Acetonitril, Propionitril oder Benzonitril); einem Acetylacetonat; einem Carboxylat (z.B. Acetat); einem Pseudohalogenid (z.B. CN⁻ oder OCN⁻), einem Amin oder einem Aryl.

Hinsichtlich des Phosphinliganden, der kein erfindungsgemäßes Phosphin ist, kann auf die obigen Ausführungen zu Ligand L² verwiesen werden. Bevorzugt ist der Phosphinligand, der kein erfindungsgemäßes Phosphin ist, ein nicht-cyclisches Phosphin, d.h. ein Phosphin, das keinen phosphorhaltigen Ring aufweist. Geeignete nicht-cyclische Phosphinliganden für Palladiumkomplexe sind dem Fachmann bekannt. Bei dem Phosphinliganden, der kein erfindungsgemäßes Phosphin ist, handelt es sich beispielsweise um ein Tri-C₁₋₆-Alkylphosphin, ein Tri-C₅₋₇-Cycloalkylphosphin oder ein Triarylphosphin (insbesondere ein Triphenylphosphin), wobei jede der Arylgruppen (die bevorzugt Phenylgruppen sind) optional durch eine oder mehrere C₁₋₄-Haloalkylgruppen (z.B. -CF₃), C₁₋₄-Alkylgruppen (z.B. Methyl) oder C₁₋₄ - Alkoxygruppen (z.B. Methoxy) substituiert ist. Alternativ kann es sich bei dem Phosphinliganden, der kein erfindungsgemäßes Phosphin ist, beispielsweise um ein Phosphin der oben beschriebenen Formel (8) handeln.

Als beispielhafte Palladiumverbindungen der erfindungsgemäßen Zusammensetzung können Folgende genannt werden: ein Palladiumdibenzylidenkomplex (z.B. Pd₂(dba)₃ oder Pd(dba)₂); PdCl₂(PR₃)₂, wobei R ein Phenyl (das optional mit einer oder mehreren C₁₋₆-Alkylgruppen substituiert ist), ein C₅₋₇-Cycloalkyl oder ein C₁₋₆-Alkyl ist; ein Palladiumacetat (z.B. Pd₂(OAc)₃); ein Palladiumacetylacetonat; ein PdX₂, wobei X ein Halogenid oder Pseudehalogenid ist; ein Pd(RCN)₂Cl₂, wobei R ein Phenyl oder Methyl ist.

Die vorliegende Erfindung betrifft außerdem die Verwendung des oben beschriebenen erfindungsgemäßen Palladiumkomplexes oder der oben beschriebenen erfindungsgemäßen Zusammensetzung als Katalysator in einer Kreuzkupplungsreaktion.

Die Kreuzkupplungsreaktion ist beispielsweise eine C-C- oder C-N-Kreuzkupplungsreaktion.

Eine bevorzugte C-N-Kreuzkupplungsreaktion ist die Buchwald-Hartwig-Kupplung. Wie dem Fachmann bekannt ist, handelt es sich bei der Buchwald-Hartwig-Kupplung um eine Kupplungsreaktion, bei der ein Aryl- oder Heteroarylhalogenid, -pseudohalogenid oder -sulfonat und ein primäres oder sekundäres Amin in Anwesenheit eines palladiumhaltigen Katalysators (und bevorzugt einer Base) unter Ausbildung einer C-N-Bindung miteinander umgesetzt werden.

Die C-C-Kreuzkupplungsreaktion ist beispielsweise eine Suzuki-Miyaura-Kupplung. Wie dem Fachmann bekannt ist, handelt es sich bei der Suzuki-Miyaura-Kupplung um eine Kupplungsreaktion, bei der eine Organobor-Verbindung und beispielsweise ein Aryl- oder Heteroarylhalogenid, -pseudohalogenid oder -sulfonat in Anwesenheit eines palladiumhaltigen Katalysators unter Ausbildung einer C-C-Bindung miteinander umgesetzt werden.

Die vorliegende Erfindung betrifft außerdem ein Verfahren zur Herstellung eines Aryl- oder Heteroarylamins, wobei eine Verbindung der Formel (9)

Ar-X (9)

wobei
Ar ein Aryl oder Heteroaryl ist,
X ein Halogenatom, eine Sulfonatgruppe (z.B. Trifluormethansulfonat -O-Tf) oder eine
Pseudohalogengruppe (z.B. -CN, -OCN oder -NCO) ist,
mit einem primären oder sekundären Amin in Anwesenheit des oben beschriebenen erfindungsgemäßen Palladiumkomplexes oder der oben beschriebenen erfindungsgemäßen Zusammensetzung umgesetzt wird.

Geeignete Reaktionsbedingungen für die Buchwald-Hartwig-Kupplung sind dem Fachmann bekannt. Bevorzugt erfolgt die Umsetzung in Anwesenheit einer Base.

### Beispiele

### Herstellung eines erfindungsgemäßen cyclischen Biarylphosphins

Ein erfindungsgemäßes cyclisches Biarylphosphin der folgenden Formel (I) 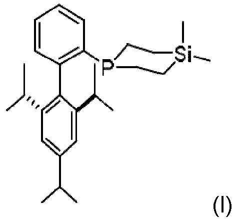 wurde nach dem folgenden Reaktionsschema hergestellt:

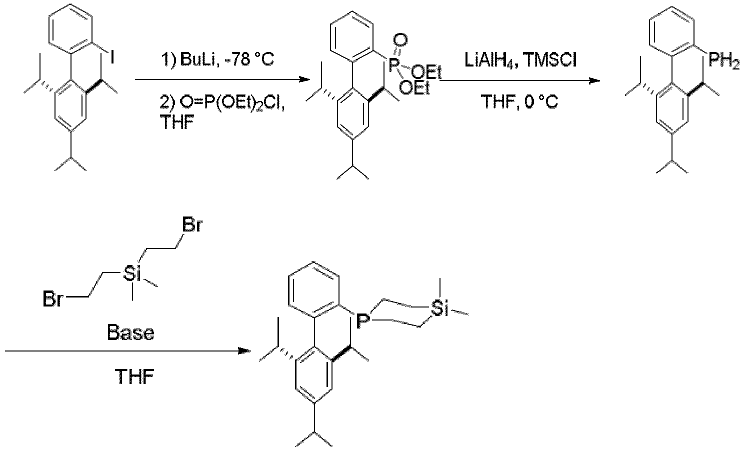

Chemischer Name des cyclischen Biarylphosphins der Formel (I): 4,4-Dimethyl-1-(2',4',6'-triisopropyl-[1,1'-biphenyl]-2-yl)-1,4-phosphasilinan Die Herstellung der Zwischenprodukte 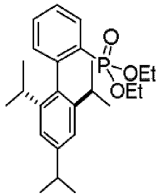
(chemischer Name: Diethyl(2',4',6'-triisopropyl-[1,1'-biphenyl]-2-yl)phosphonat) und 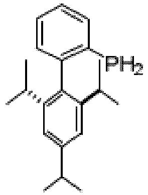
(chemischer Name: 2',4',6'-Triisopropyl-[1,1'-biphenyl]-2-yl)phosphan) erfolgte nach den von S. Shekhar et al., ACS Catal., 2019, 9, S. 11691-11708, beschriebenen Synthesen.

### Diethyl(2',4',6'-triisopropyl-[1,1'-biphenyl]-2-yl)phosphonat:

2'-Iodo-2,4,6-triisopropyl-1,1'-biphenyl (438 mg; 1,08 mmol; 1,00 equiv.) unter Argon wurde in entgastem, trockenem THF (5 mL) gelöst und n-BuLi (2,50 M in Hexan; 0,47 mL; 1,10 mmol; 1,10 equiv.) bei -78 °C über 30 Minuten langsam hinzugetropft. Die Lösung wurde bei -78 °C für 30 Minuten gerührt. Diethylchlorphosphat (0,19 mL; 1,29 mmol; 1,20 equiv.) wurden bei -78 °C hinzugefügt und die Lösung weitere 30 Minuten bei -78 °C gerührt. Anschließend wurde die Reaktionslösung für 5 Stunden bei Raumtemperatur gerührt. Die Reaktion wurde mit einer wässrigen, gesättigten Lösung von NH₄Cl (7 mL) gequencht, abfiltriert und die wässrige Phase mit EtOAc (3 × 7 mL) extrahiert. Die vereinten organischen Phasen wurden mit gesättigter Kochsalzlösung (7 mL) gewaschen und über Na₂SO₄ getrocknet. Das Lösungsmittel wurde *in vacuo* entfernt. Der Rückstand wurde durch Säulenchromatographie (Kieselgel, Hexan/EtOAc: 0-60 % EtOAc) aufgereinigt. Das Produkt (335 mg; 0,80 mmol; 75 %) wurde als farbloser Feststoff erhalten.

### (2',4',6'-Triisopropyl-[1,1'-biphenyl]-2-yl)phosphan:

LiAlH₄ (1,00 M in THF; 5,04 mL; 5.04 mmol; 3,00 equiv.) wurde unter Argon bei 0 °C in entgastes, trockenes THF (5 mL) gegeben. Trimethylchlorsilan (0,64 mL; 5,04 mmol; 3,00 equiv.) wurde bei 0 °C hinzugegeben und die Lösung für 30 Minuten bei 0 °C gerührt. Die Lösung wurde zu einer Lösung von Diethyl (2',4',6'-triisopropyl-[1,1'-biphenyl]-2-yl)phosphonat (335 mg; 0,80 mmol; 1,00 equiv.) in THF (5 mL) bei 0 °C hinzugetropft. Die Reaktionslösung wurde über Nacht bei Raumtemperatur gerührt, anschließend auf 0 °C gekühlt und mit EtOAc (10 mL) und einer wässriger 1M HCl-Lösung (26 mL) gequencht. Das Reaktionsgemisch wurde für eine Stunde gerührt und die wässrige Phase mit EtOAc (2 × 10 mL) extrahiert. Die vereinten organischen Phasen wurden mit gesättigter Kochsalzlösung (10 mL) gewaschen, über Na₂SO₄ getrocknet und das Lösungsmittel *in vacuo* entfernt. Das Produkt (239 mg; 0,76 mmol; 95 %) wurde als farbloser Feststoff erhalten.

### 4,4-Dimethyl-1-(2',4',6'-triisopropyl-[1,1'-biphenyl]-2-yl)-1,4-phosphasilinan:

(2',4',6'-Triisopropyl-[1,1'-biphenyl]-2-yl)phosphan (200 mg; 0,64 mmol; 1,00 equiv.) wurden in trockenem, entgastem THF (3 mL) gelöst. n-BuLi (2,5 M in Hexan; 0,51 mL; 1,28 mmol; 2,00 equiv.) wurde bei -78 °C hinzugetropft. Es wurde für 10 Minuten bei Raumtemperatur gerührt. Anschließend wurde Bis(bromoethyl)dimethylsilan (175 mg; 0,64 mmol; 1,00 equiv.) bei -78 °C hinzugegeben. Die Reaktion wurde für 4 Stunden bei Raumtemperatur gerührt. Die Reaktion wurde mit einer entgasten, wässrigen, gesättigten Lösung von NH₄Cl (5 mL) gequencht. Die wässrige Phase wurde mit entgastem, trockenem EtOAc (3 × 7 mL) gewaschen und die vereinten organischen Phasen über Na₂SO₄ getrocknet. Das Lösungsmittel wurde *in vacuo* entfernt. Das Produkt (259 mg; 0,61 mmol; 95 %) konnte als farblose, viskose Flüssigkeit isoliert werden.

### Verwendung von Zusammensetzungen, die ein Biarylphosphin und eine Palladiumverbindung enthalten, als Katalysator in einer Buchwald-Hartwig-Kupplung

In den nachfolgend beschriebenen erfindungsgemäßen Beispielen 1-13 wurde das cyclische Biarylphosphin der Formel (I) verwendet, d.h.:

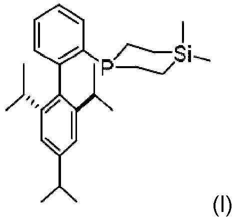

In dem Vergleichsbeipiel 1 wurde ein nicht-cyclisches Biarylphosphin der folgenden Formel (II) verwendet:

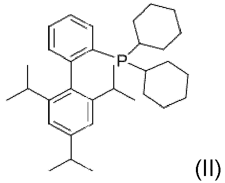

Als Palladiumverbindung wurde in allen Beispielen (d.h. den erfindungsgemäßen Beispielen 1-13 und dem Vergleichsbeispiel 1) ein Palladiumdibenzylidenkomplex (Pd₂(dba)₃) verwendet. Diese Palladiumverbindung und das Phosphin der Formel (I) oder (II) wurden dem Reaktionsmedium zugegeben, so dass sich *in situ* ein Palladiumkomplex, der das Phosphin als einen seiner Liganden enthält, bilden konnte.

In Beispiel 1 wurden 2-Chlorchinolin und Piperidin als Reaktanten für die Buchwald-Hartwig Kupplung verwendet.

### Beispiel 1:

2-Chlorochinolin (150 mg; 0,92 mmol; 1,00 equiv.) wurde in trockenem, entgastem Toluol (6 mL) gelöst. Pd₂(dba)₃ (16,8 mg; 0,02 mmol; 0,02 equiv.), NaOtBu (123 mg; 1,28 mmol; 1,40 equiv.), das cyclische Biarylphosphin der Formel (I) (0,5 M in Toluol; 0,07 mL; 0,04 mmol; 0,04 equiv.) und Piperidin (109 mL; 1,10 mmol; 1,20 equiv.) wurden hinzugegeben. Die Reaktion wurde bei 60 °C für 2 Stunden gerührt und anschließend mit einer wässrigen, gesättigten Lösung von NH₄Cl gequencht. Die wässrige Phase wurde mit EtOAc (3 × 8 mL) gewaschen und die vereinten organischen Phasen über Na₂SO₄ getrocknet. Das Lösungsmittel wurde *in vacuo* entfernt. Das Produkt wurde durch Säulenchromatograpie (Kieselgel, Hexan/EtOAc: 20/1) aufgereinigt. 2-(Piperidin-1-yl)chinolin (173 mg; 0,81 mmol; 89 %) konnte als farbloser Feststoff isoliert werden.

In den erfindungsgemäßen Beispielen 2-13 wurden die Reaktanten variiert (siehe nachfolgende Tabelle 1), die Synthesebedingungen waren jedoch identisch mit den in Beispiel 1 verwendeten Synthesebedingungen.

In Vergleichsbeispiel 1 waren die Reaktanten und die Synthesebedingungen identisch mit den in Beispiel 1 verwendeten Reaktanten und Synthesebedingungen. Allerdings wurde anstelle des erfindungsgemäßen Phosphins der Formel (I) das Phosphin der Formel (II) verwendet.

Die Ergebnisse der erfindungsgemäßen Beispiele 1-13 sind in der nachfolgenden Tabelle 1 zusammengefasst.

**Tabelle 1: Als Reaktanten verwendete Amine, bei der Buchwald-Hartwig-Kupplung erhaltene Reaktionsprodukte und Produktausbeuten in den erfindungsgemäßen Beispielen 1-13**

| Beispiel | Heteroaryl | Amin | Reaktionsprodukt | Ausbeute [%] |
|---|---|---|---|---|
| 1 | 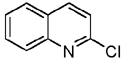 | 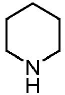 | 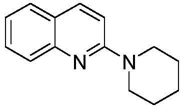 | 88 |
| 2 | 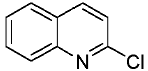 | 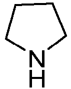 | 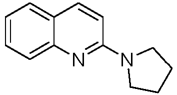 | 74 |
| 3 | 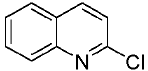 | 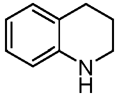 | 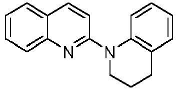 | 100 |
| 4 | 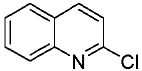 | 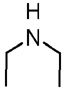 | 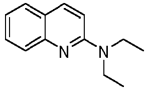 | 42 |
| 5 | 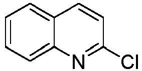 | 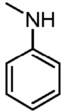 | 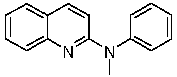 | 86 |
| 6 | 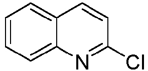 | 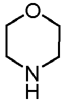 | 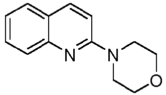 | 95 |
| 7 | 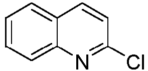 | 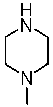 | 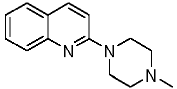 | 67 |
| 8 | 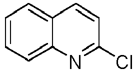 | 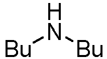 | 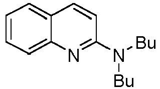 | 79 |
| 9 | 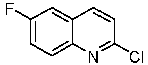 | 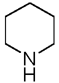 | 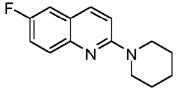 | 82 |
| 10 | 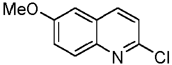 | 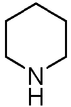 | 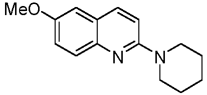 | 100 |
| 11 | 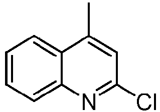 | 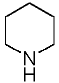 | 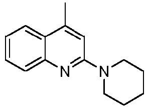 | 98 |
| 12 | 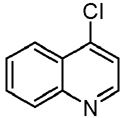 | 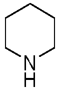 | 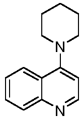 | 40 |
| 13 | 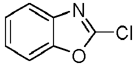 | 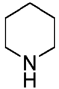 | 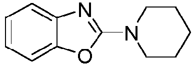 | 86 |

Obwohl in allen Beispielen einer der Reaktanten ein N-heterocyclisches Arylhalogenid war und eine recht kurze Reaktionsdauer bei relativ milder Reaktionstemperatur gewählt wurde, führte die Verwendung des erfindungsgemäßen cyclischen Biarylphosphins als Ligand eines Palladiumkomplexes bei einer palladiumkatalysierten Buchwald-Hartwig-Kupplung zu hohen Produktausbeuten.

Das Ergebnis des Vergleichsbeispiels 1 ist in der nachfolgenden Tabelle 2 angegeben.

**Tabelle 2: Als Reaktanten verwendete Amine und Produktausbeute bei der Buchwald-Hartwig-Kupplung im Vergleichsbeispiel 1**

| | Heteroaryl | Amin | Erwünschtes Reaktionsprodukt | Ausbeute |
|---|---|---|---|---|
| Vergl.bsp. 1 | 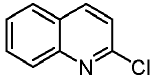 | 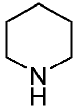 | 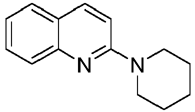 | 0% |

### Verwendung einer Zusammensetzung, die ein erfindungsgemäßes cyclisches Biarylphosphin und eine Palladiumverbindung enthält, als Katalysator in einer Suzuki-Miyaura-Kupplung

Unter Verwendung eines erfindungsgemäßen cyclischen Biarylphosphins der Formel (I) wurde in den erfindungsgemäßen Beispielen 14-17 eine Suzuki-Miyaura-Kupplung mit den im nachfolgenden Reaktionsschema und in der Tabelle 3 angegebenen Reaktanten und Reaktionsbedingungen durchgeführt. Die Ausbeuten sind in der Tabelle 3 angegeben. 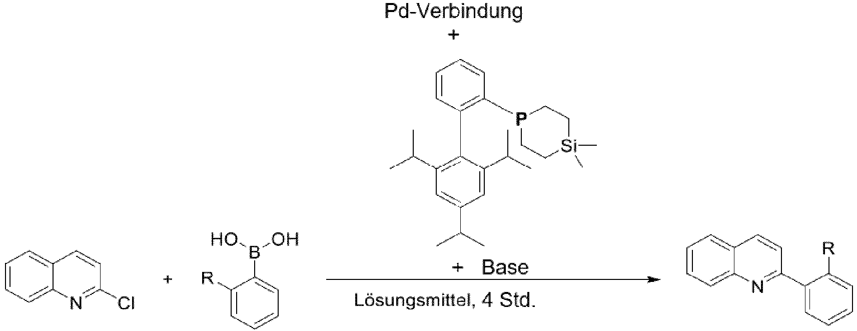 R = H, Me

In Beispiel 14 erfolgte die Herstellung von 2-(o-Tolyl)chinolin (d.h. R=Me) folgendermaßen:
2-Chlorochinolin (150 mg; 0,92 mmol; 1,00 equiv.) wurde in trockenem, entgastem THF (5 mL) und entgastem Wasser (1mL) gelöst. Pd₂(dba)₃ (16,8 mg; 0,02 mmol; 0,02 equiv.), CsOH × H₂O (216 mg; 1,28 mmol; 1,40 equiv.), das cyclische Biarylphosphin der Formel (I) 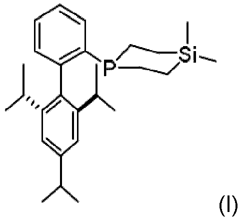 (0,5 M in Toluol; 0,07 mL; 0,04 mmol; 0,04 equiv.) und o-Tolylboronsäure (150 mg; 1,10 mmol; 1,20 equiv.) wurden hinzugegeben. Die Reaktion wurde bei 60 °C für 4 Stunden gerührt und anschließend mit einer wässrigen, gesättigten Lösung von NH₄Cl gequencht. Die wässrige Phase wurde mit EtOAc (3 × 8 mL) gewaschen und die vereinten organischen Phasen über Na₂SO₄ getrocknet. Das Lösungsmittel wurde *in vacuo* entfernt. Das Produkt wurde durch Säulenchromatograpie (Kieselgel, Hexan/EtOAc: 10/1) aufgereinigt. 2-(o-Tolyl)chinolin (179 mg; 0,82 mmol; 89 %) konnte als farbloser Feststoff isoliert werden.

Die Suzuki-Miyaura-Kupplung in den erfindungsgemäßen Beispielen 15-17 basierte auf Beispiel 14, allerdings mit den aus der Tabelle 3 ersichtlichen Variationen der Synthesebedingungen.

In jedem der Beispiele 14-17 wurden somit dem Reaktionsmedium das cyclische Biarylphosphin der Formel (I) und eine Palladiumverbindung (Pd₂(dba)₃) oder Pd(OAc)₂) zugegeben, so dass sich *in situ* ein Palladiumkomplex, der das erfindungsgemäße cyclische Biarylphosphin als einen seiner Liganden enthält, bilden konnte.

**Tabelle 3: Reaktionsausbeuten in den Beispielen 14-17**

| | Pd-Verbindung | R | Base | Lösungsmittel | Reaktionstemperatur | Ausbeute |
|---|---|---|---|---|---|---|
| Beispiel 14 | Pd₂(dba)₃ | Me | CsOH x H₂O | THF/H₂O | 60°C | 89 % |
| Beispiel 15 | Pd₂(dba)₃ | H | NaOtBu | THF/H₂O | Raumtemperatur | 35 % |
| Beispiel 16 | Pd₂(dba)₃ | H | CsOH x H₂O | THF/H₂O | Raumtemperatur | 54 % |
| Beispiel 17 | Pd(OAc)₂ | H | CsOH x H₂O | THF/H₂O | Raumtemperatur | 46 % |

Obwohl in allen Beispielen einer der Reaktanten ein N-heterocyclisches Arylhalogenid war und eine recht kurze Reaktionsdauer (4 Std.) bei milder Reaktionstemperatur gewählt wurde, führte die Verwendung des erfindungsgemäßen cyclischen Biarylphosphins als Ligand eines Palladiumkomplexes bei einer palladiumkatalysierten Suzuki-Miyaura-Kupplung zu hohen Produktausbeuten.

## Patentansprüche

1. Ein Phosphin der Formel (1) 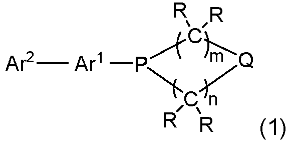 wobei
- Q SiR³R⁴ ist;
- m 1, 2 oder 3 ist; n 1, 2 oder 3 ist; unter der Maßgabe, dass folgende Beziehung erfüllt ist: 3 ≤ m + n ≤ 5;
- die Reste R unabhängig voneinander ein Wasserstoffatom oder ein C₁₋₄-Alkyl sind; oder zwei Reste R, die mit demselben Kohlenstoffatom verbunden sind, jeweils eine bivalente Alkylengruppe sind und zusammen mit dem Kohlenstoffatom, mit dem sie verbunden sind, einen 4- bis 7-gliedrigen Ring bilden; oder zwei Reste R, die mit unterschiedlichen Kohlenstoffatomen verbunden sind, jeweils eine bivalente Alkylengruppe sind und zusammen mit den Kohlenstoffatomen, mit denen sie verbunden sind, einen 4- bis 7-gliedrigen Ring bilden;
- Ar¹ ein Phenyl oder Naphthyl ist, das optional mit einer oder mehreren C₁₋₆-Alkylgruppen substituiert ist;
- Ar² ein Phenyl oder Naphthyl ist, das optional mit einer oder mehreren C₁₋₆-Alkylgruppen substituiert ist;
- R³ und R⁴ unabhängig voneinander ein C₁₋₄-Alkyl oder ein Phenyl, das optional mit einer oder mehreren C₁₋₆-Alkylgruppen substituiert ist, sind.

2. Das Phosphin nach Anspruch 1, die folgende Formel (1a) aufweisend: 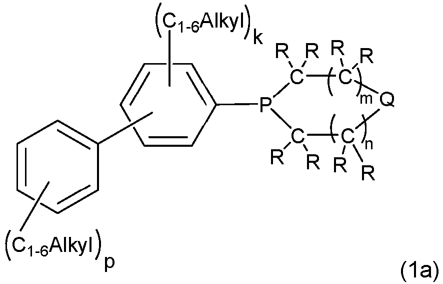 wobei
m 0 oder 1 (bevorzugter m=1) und n 1 oder 2 (bevorzugter n=1) sind;
k 0, 1, 2, 3 oder 4 ist;
p 0, 1, 2, 3, 4 oder 5 ist;
Q und R jeweils die in Anspruch 1 angegebene Bedeutung aufweisen.

3. Das Phosphin nach einem der Ansprüche 1 bis 2, folgende Formel (1b) aufweisend: 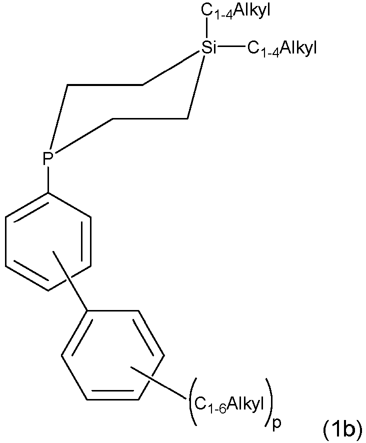 wobei
p 0, 1, 2, 3, 4 oder 5, bevorzugter 1, 2, 3 oder 4 ist.

4. Ein Verfahren zur Herstellung des Phosphins gemäß einem der Ansprüche 1 bis 3, wobei ein Phosphin der Formel (5)
Ar¹-Ar²-PH₂ (5)
wobei
Ar¹ und Ar² jeweils die in einem der Ansprüche 1-3 angegebene Bedeutung aufweisen,
mit einer Base zumindest teilweise deprotoniert wird und das dabei erhaltene Phosphid umgesetzt wird mit einer Verbindung der Formel (6) 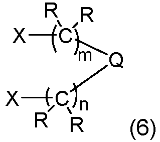 wobei
X ein Halogenatom ist,
Q, R, m und n jeweils die in einem der Ansprüche 1-3 angegebene Bedeutung aufweisen.

5. Das Verfahren nach Anspruch 4, wobei das Phosphin der Formel (5) hergestellt wird, indem eine Verbindung der Formel (4) 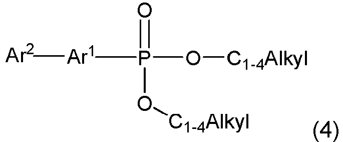 wobei
Ar¹ und Ar² jeweils die in einem der Ansprüche 1-3 angegebene Bedeutung aufweisen;
in Anwesenheit eines Reduktionsmittels reduziert wird.

6. Das Verfahren nach Anspruch 4 oder 5, wobei die Verbindung der Formel (4) hergestellt wird, indem eine Verbindung der Formel (2)
Ar¹-Ar²-X (2)
wobei
X ein Halogenatom ist,
Ar¹ und Ar² jeweils die in einem der Ansprüche 1-3 angegebene Bedeutung aufweisen,
mit einer metallorganischen Verbindung, bevorzugt einer Organolithiumverbindung, zu einem Zwischenprodukt umgesetzt wird und das Zwischenprodukt anschließend umgesetzt wird mit einer Verbindung der Formel (3)
O=P(O-C₁₋₄-Alkyl)₂X (3)
wobei X ein Halogenatom ist.

7. Ein Palladiumkomplex, enthaltend als einen Liganden L¹ das Phosphin gemäß einem der Ansprüche 1 bis 3.

8. Der Palladiumkomplex nach Anspruch 7, zusätzlich enthaltend einen oder mehrere Liganden L², die jeweils kein Phosphin gemäß einem der Ansprüche 1 bis 3 sind.

9. Der Palladiumkomplex nach Anspruch 8, wobei die Liganden L² unabhängig voneinander ausgewählt sind aus einem Halogenid; einem Aryl; einem Nitril; einem Carboxylat; einem konjugierten Dienon; einem Phosphin, das kein Phosphin nach einem der Ansprüche 1 bis 3 ist; einem Amin oder Acetylacetonat.

10. Eine Zusammensetzung, enthaltend
- eine Palladiumverbindung und
- das Phosphin gemäß einem der Ansprüche 1-3.

11. Die Zusammensetzung nach Anspruch 10, wobei die Palladiumverbindung ein Palladiumsalz oder ein Palladiumkomplex, der kein Phosphin gemäß einem der Ansprüche 1-3 als Liganden enthält, ist.

12. Die Zusammensetzung nach Anspruch 10 oder 11, wobei das Palladiumsalz ein Palladiumacetat, ein Palladiumhalogenid, ein Palladiumpseudohalogenid oder ein Gemisch aus mindestens zwei dieser Salze ist.

13. Die Zusammensetzung nach Anspruch11, wobei die Liganden des Palladiumkomplexes unabhängig voneinander ausgewählt sind aus einem Halogenid; einem Phosphin, das kein Phosphin gemäß einem der Ansprüche 1 bis 3 ist; einem konjugierten Dienon; einem Polyen; einem Nitril; einem Acetylacetonat; einem Carboxylat; einem Pseudohalogenid; einem Amin oder einem Aryl.

14. Verwendung des Palladiumkomplexes gemäß einem der Ansprüche 7 bis 9 oder der Zusammensetzung gemäß einem der Ansprüche 10 bis 13 als Katalysator in einer Kreuzkupplungsreaktion.

15. Die Verwendung nach Anspruch14, wobei die Kreuzkupplungsreaktion eine C-C- oder eine C-N-Kreuzkupplungsreaktion, insbesondere eine Buchwald-Hartwig-Kupplung ist.

16. Ein Verfahren zur Herstellung eines Aryl- oder Heteroarylamins, wobei eine Verbindung der Formel (9)
Ar-X (9)
wobei
Ar ein Aryl oder Heteroaryl ist,
X ein Halogenatom, eine Sulfonatgruppe oder eine Pseudohalogengruppe ist,
mit einem primären oder sekundären Amin in Anwesenheit des Palladiumkomplexes gemäß einem der Ansprüche 7 bis 9 oder der Zusammensetzung gemäß einem der Ansprüche 10 bis 13 umgesetzt wird.

## Claims

1. A phosphine of formula (1) 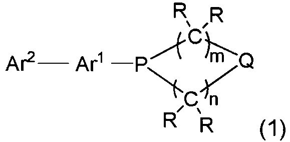 wherein
- Q is SiR³R⁴;
- m is 1, 2 or 3; n is 1, 2 or 3; under the proviso that the following relationship is met: 3 ≤ m + n ≤ 5;
- the groups R are, independently of one another, a hydrogen atom or a C₁₋₄ alkyl; or two groups R that bind to the same carbon atom are in each case a divalent alkylene group and together with the carbon atom to which they are bound form a 4- to 7-membered ring; or two groups R that bind to different carbon atoms are in each case a divalent alkylene group and together with the carbon atoms to which they are bound form a 4- to 7-membered ring;
- Ar¹ is a phenyl or naphthyl optionally substituted with one or more C₁₋₆ alkyl groups;
- Ar² is a phenyl or naphthyl optionally substituted with one or more C₁₋₆ alkyl groups;
- R³ and R⁴ are, independently of one another, a C₁₋₄ alkyl or a phenyl optionally substituted with one or more C₁₋₆ alkyl groups.

2. The phosphine according to claim 1, having the following formula (1a): 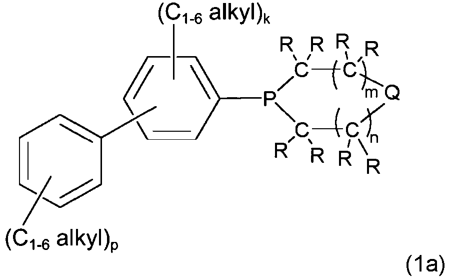 wherein
m is 0 or 1 (more preferably m = 1) and n is 1 or 2 (more preferably n = 1);
k is 0, 1, 2, 3 or 4;
p is 0, 1, 2, 3, 4, or 5;
Q and R each have the meaning indicated in claim 1.

3. The phosphine according to any one of claims 1 to 2, having the following formula (1b): 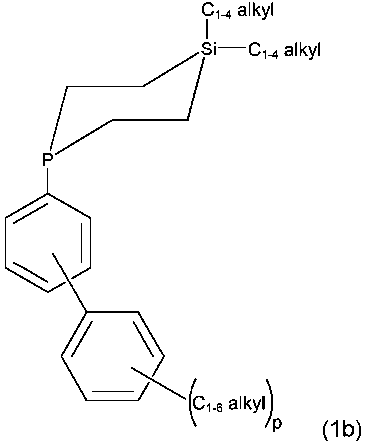 wherein
p is 0, 1, 2, 3, 4 or 5, more preferably 1, 2, 3 or 4.

4. A method for preparing the phosphine according to any one of claims 1 to 3, wherein a phosphine of formula (5)
Ar¹-Ar²-PH₂ (5)
wherein
Ar¹ and Ar² each have the meaning indicated in any one of claims 1-3, is at least partially deprotonated with a base and the resulting phosphide is reacted with a compound of formula (6) 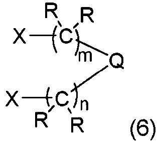
wherein
X is a halogen atom,
Q, R, m and n each have the meaning indicated in any one of claims 1-3.

5. The method according to claim 4, wherein the phosphine of formula (5) is prepared by reducing a compound of formula (4) 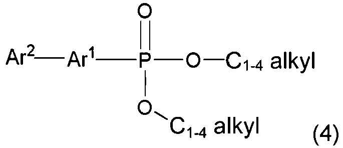 wherein
Ar¹ and Ar² each have the meaning indicated in any one of claims 1-3; in the presence of a reducing agent.

6. The method according to claim 4 or 5, wherein the compound of formula (4) is prepared by reacting a compound of formula (2)
Ar¹-Ar²-X (2)
wherein
X is a halogen atom,
Ar¹ and Ar² each have the meaning indicated in any one of claims 1-3,
with an organometallic compound, preferably an organolithium compound, to form an intermediate product, and by subsequently reacting the intermediate product with a compound of formula (3)
O=P(O-C₁₋₄ alkyl)₂X (3)
wherein X is a halogen atom.

7. A palladium complex containing the phosphine according to any one of claims 1 to 3 as a ligand L¹.

8. The palladium complex according to claim 7, additionally containing one or more ligands L², each of which is not a phosphine according to any one of claims 1 to 3.

9. The palladium complex according to claim 8, wherein the ligands L² are independently selected from a halide; an aryl; a nitrile; a carboxylate; a conjugated dienone; a phosphine which is not a phosphine according to any one of claims 1 to 3; an amine or acetylacetonate.

10. A composition containing
- a palladium compound, and
- the phosphine according to any one of claims 1-3.

11. The composition according to claim 10, wherein the palladium compound is a palladium salt or a palladium complex that does not contain a phosphine according to any one of claims 1-3 as a ligand.

12. The composition according to claim 10 or 11, wherein the palladium salt is a palladium acetate, a palladium halide, a palladium pseudohalide, or a mixture of at least two of these salts.

13. The composition according to claim 11, wherein the ligands of the palladium complex are independently selected from a halide; a phosphine which is not a phosphine according to any one of claims 1 to 3; a conjugated dienone; a polyene; a nitrile; an acetylacetonate; a carboxylate; a pseudohalide; an amine or an aryl.

14. A use of the palladium complex according to any one of claims 7 to 9 or the composition according to any one of claims 10 to 13 as a catalyst in a cross-coupling reaction.

15. The use according to claim 14, wherein the cross-coupling reaction is a C-C or a C-N cross-coupling reaction, in particular a Buchwald-Hartwig coupling.

16. A method for preparing an arylamine or heteroarylamine, wherein a compound of formula (9)
Ar-X (9)
wherein
Ar is an aryl or heteroaryl,
X is a halogen atom, a sulfonate group, or a pseudohalogen group, is reacted with a primary or secondary amine in the presence of the palladium complex according to any one of claims 7 to 9 or the composition according to any one of claims 10 to 13.

## Revendications

1. Phosphine de formule (1) 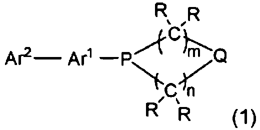 où
- Q représente SiR³R⁴ ;
- m représente 1, 2 ou 3 ; n représente 1, 2 ou 3 ; à condition que la relation suivante soit remplie : 3 ≤ m + n ≤ 5 ;
- les radicaux R représentent, indépendamment les uns des autres, un atome d'hydrogène ou un alkyle en C₁₋₄ ; ou deux radicaux R liés au même atome de carbone représentent respectivement un groupe alkylène divalent et forment, avec l'atome de carbone auquel ils sont liés, un cycle de 4 à 7 chaînons ; ou deux radicaux R liés à différents atomes de carbone représentent respectivement un groupe alkylène divalent et forment, avec les atomes de carbone auxquels ils sont liés, un cycle de 4 à 7 chaînons ;
- Ar¹ représente un phényle ou un naphtyle éventuellement substitué par un ou plusieurs groupes alkyle en C₁₋₆ ;
- Ar² représente un phényle ou un naphtyle éventuellement substitué par un ou plusieurs groupes alkyle en C₁₋₆ ;
- R³ et R⁴ représentent, indépendamment l'un de l'autre, un alkyle en C₁₋₄ ou un phényle éventuellement substitué par un ou plusieurs groupes alkyle en C₁₋₆.

2. Phosphine selon la revendication 1, présentant la formule (1a) suivante : 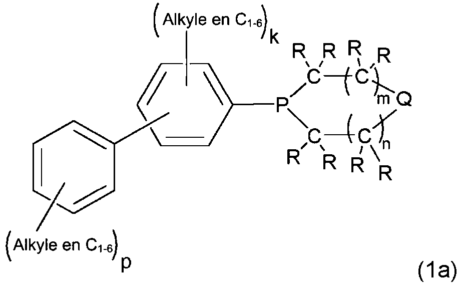 où
m représente 0 ou 1 (de préférence m = 1) et n représente 1 ou 2 (de préférence n = 1) ;
k représente 0, 1, 2, 3 ou 4 ;
p représente 0, 1, 2, 3, 4 ou 5 ;
Q et R présentent respectivement la signification donnée dans la revendication 1.

3. Phosphine selon l'une des revendications 1 à 2, présentant la formule (1b) suivante : 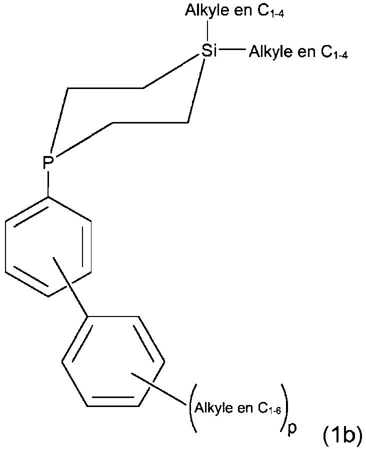 où
p représente 0, 1, 2, 3, 4 ou 5, de préférence 1, 2, 3 ou 4.

4. Procédé permettant la préparation de la phosphine selon l'une des revendications 1 à 3, dans lequel une phosphine de formule (5)
Ar¹-Ar²-PH₂ (5)
où
Ar¹ et Ar² présentent respectivement la signification donnée dans l'une des revendications 1 à 3,
est au moins partiellement déprotonée avec une base et le phosphure ainsi obtenu est mis à réagir avec un composé de formule (6) 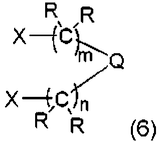 où
X représente un atome d'halogène,
Q, R, m et n présentent respectivement la signification donnée dans l'une des revendications 1 à 3.

5. Procédé selon la revendication 4, dans lequel la phosphine de formule (5) est préparée par le fait qu'un composé de formule (4) 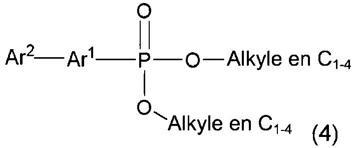 où
Ar¹ et Ar² présentent respectivement la signification donnée dans l'une des revendications 1 à 3 ;
est réduit en présence d'un agent de réduction.

6. Procédé selon la revendication 4 ou 5, dans lequel le composé de formule (4) est préparé par le fait qu'un composé de formule (2)
Ar¹-Ar²-X (2)
où
X représente un atome d'halogène,
Ar¹ et Ar² présentent respectivement la signification donnée dans l'une des revendications 1 à 3,
est mis à réagir avec un composé organométallique, de préférence un composé organolithien, pour obtenir un produit intermédiaire, et le produit intermédiaire est ensuite mis à réagir avec un composé de formule (3)
O=P(O-alkyle en C₁₋₄)₂X (3)
où X représente un atome d'halogène.

7. Complexe de palladium, contenant comme ligand L¹ la phosphine selon l'une des revendications 1 à 3.

8. Complexe de palladium selon la revendication 7, contenant en outre un ou plusieurs ligands L² qui ne sont respectivement pas une phosphine selon l'une des revendications 1 à 3.

9. Complexe de palladium selon la revendication 8, dans lequel les ligands L² sont choisis indépendamment les uns des autres parmi un halogénure ; un aryle ; un nitrile ; un carboxylate ;
une diénone conjuguée ; une phosphine autre qu'une phosphine selon l'une des revendications 1 à 3 ; une amine ou de l'acétylacétonate.

10. Composition, contenant
- un composé de palladium et
- la phosphine selon l'une des revendications 1 à 3.

11. Composition selon la revendication 10, dans laquelle le composé de palladium est un sel de palladium ou un complexe de palladium qui ne contient pas de phosphine selon l'une des revendications 1 à 3 comme ligand.

12. Composition selon la revendication 10 ou 11, dans laquelle le sel de palladium est un acétate de palladium, un halogénure de palladium, un pseudo-halogénure de palladium ou un mélange d'au moins deux de ces sels.

13. Composition selon la revendication 11, dans laquelle les ligands du complexe de palladium sont choisis indépendamment les uns des autres parmi un halogénure ; une phosphine autre qu'une phosphine selon l'une des revendications 1 à 3 ; une diénone conjuguée ; un polyène ; un nitrile ; un acétylacétonate ; un carboxylate ; un pseudo-halogénure ; une amine ou un aryle.

14. Utilisation du complexe de palladium selon l'une des revendications 7 à 9 ou de la composition selon l'une des revendications 10 à 13 comme catalyseur dans une réaction de couplage croisé.

15. Utilisation selon la revendication 14, dans laquelle la réaction de couplage croisé est une réaction de couplage croisé C-C ou C-N, en particulier un couplage Buchwald-Hartwig.

16. Procédé permettant la préparation d'une arylamine ou d'une hétéroarylamine, dans lequel un composé de formule (9)
Ar-X (9)
où
Ar représente un aryle ou un hétéroaryle,
X représente un atome d'halogène, un groupe sulfonate ou un groupe pseudohalogène,
est mis à réagir avec une amine primaire ou secondaire en présence du complexe de palladium selon l'une des revendications 7 à 9 ou de la composition selon l'une des revendications 10 à 13.
